# EUROPÄISCHE PATENTSCHRIFT

(11) **EP 1 627 608 B1**
(45) Veröffentlichungstag und Bekanntmachung des Hinweises auf die Patenterteilung: **06.05.2009**
(21) Anmeldenummer: 04405521.8
(22) Anmeldetag: 20.08.2004
(51) Int. Cl.: A61B 17/64

(54) **Klemmelement und Gelenkelement**
Clamp element and joint element
Elément de serrage et élément de joint

(43) Veröffentlichungstag der Anmeldung: 22.02.2006
(73) Patentinhaber: Stryker Trauma SA, 2545 Selzach (CH)
(72) Erfinder: Thomke, Roland, 4512 Bellach (CH); Burgherr, Vinzenz, 3005 Bern (CH); Lutz, Christian, 4500 Solothurn (CH); Fankhauser, Damian, 3007 Bern (CH); Dransfeld, Clemens, 5702 Niederlenz (CH); Fischer, René, 8057 Zürich (CH)
(74) Vertreter: Liebetanz, Michael

(56) Entgegenhaltungen:
- DE-A- 19 854 347
- US-A- 4 483 334
- US-A1- 2002 077 629
- US-B1- 6 277 069
- US-B2- 6 616 664

## Beschreibung

### Technisches Gebiet der Erfindung

Die Erfindung betrifft ein Klemmelement zum Klemmen eines stabförmigen Elementes einer Gelenkverbindung, insbesondere ein Klemmelement einer Gelenkverbindung zur Stabilisierung von Knochenbrüchen. Weiterhin betrifft die Erfindung eine Gelenkverbindung mit zwei Klemmelementen und mit einem mindestens zweiteiligen Verriegelungselement.

### Stand der Technik

Die EP 1 184 000 beschreibt ein einstückiges Klemmelement, das über zwei gegenüberliegende und einen seitlich offenen Hohlraum zur Aufnahme eines stabförmigen Elementes bildende Klemmbacken und ein Scharniermittel verfügt, welches gegenüber dem Hohlraum angeordnet ist, die Klemmbacken miteinander verbindet und dadurch diese aufeinander zu bewegbar sind, wobei jede Klemmbacke jeweils eine Bohrung aufweist, die miteinander fluchtend ausgerichtet sind.

Dieses Klemmelement hat den Vorteil, dass mit zwei nebeneinander angeordneten identischen Klemmelementen eine Gelenkverbindung hergestellt werden kann, wobei dabei durch die genannten Bohrungen eine Verbindungsschraube eingesetzt wird, die in eine innengewindete Mutter eingeschraubt wird, um die Klemmbacken zu schliessen.

Es ist ein Nachteil der bekannten Vorrichtung, dass die stabförmigen Elemente nur von ihren Enden her in Längsrichtung in die aufnehmenden Hohlräume eingeführt werden können.

Aus der US 6,277,069 und der US 2002/0077629 A sind andere Klemmelemente bekannt, welche seitlich offen sind. Das Klemmelement nach US 6,277,069 gestattet das seitliche Einlegen eines ersten stabförmigen Elementes. Ein zweites stabförmiges Element ist in eine mit dem Spannhebel verbundenen geschlossenen Hülse einführbar. Der Oberbegriff des Anspruchs 1 basiert auf diesen Stand der Technik.

Aus der US 6,616,664 und der EP 0 700 664 ist jeweils eine Gelenkverbindung bekannt, die aus vier einzelnen Klemmbackenelementen und einer zentralen Schraube besteht. Bei dieser Gelenkverbindung ist es möglich, einen oder zwei stabförmige Elemente in die entsprechenden Hohlräume seitlich einzuführen. Bei der EP 0 700 664 ist zwischen den beiden mittleren Klemmbackenelementen eine Feder angeordnet, gegen deren Federkraft es möglich ist, die stabförmigen Elemente einzuklipsen und so vor einer Blockierung der Gelenkverbindung diese an den stabförmigen Elemente festzuhalten. Bei der US 6,616,664 sind schmale, seitlich angeordnete Hebelarme vorgesehen, um seitlich eingelegte stabförmige Elemente vor einer Blockierung der Gelenkverbindung festzuhalten.

Ausgehend von diesem Stand der Technik ist es eine Aufgabe der vorliegenden Erfindung ein einfaches einstückiges Klemmelement anzugeben, welches das seitliche Einlegen von einem stabförmigen Element ermöglicht und welches doppelt eingesetzt direkt als Gelenkverbindung einsetzbar ist.

Ein weiteres Ziel der Erfindung ist es, ein kostengünstiges Einwegklemmelement zu schaffen, insbesondere aus Kunststoff im Spritzguss, welches nicht die strukturellen Nachteile von röntgentransparenten Klemmelementen wie nach der EP 1 184 000 aufweist.

Ausgehend vom bekannten Stand der Technik liegt der Erfindung weiterhin die Aufgabe zugrunde, eine verbesserte Gelenkverbindung anzugeben.

### Zusammenfassung der Erfindung

Diese Aufgabe wird erfindungsgemäss für ein Klemmelement der eingangs genannten Art mit den kennzeichnenden Merkmalen des Anspruchs 1 gelöst. Eine erfindungsgemässe Gelenkverbindung ist durch die Merkmale des Anspruchs 11 gekennzeichnet.

Dadurch, dass Ausführungen der einstückigen Klemmelemente mit funktionell unterschiedlichen ersten und zweiten Klemmbacken ausgestattet werden können, können zwei Klemmelemente mit ihren jeweils ersten Klemmbacken aufeinander gelegt werden, um in einfacher Weise ein Gelenkelement zu bilden.

Weitere vorteilhafte Ausführungsbeispiele sind in den Unteransprüchen niedergelegt.

### Kurze Figurenbeschreibung

Die Erfindung wird nun unter Bezugnahme auf die Zeichnungen an Hand von Ausführungsbeispielen beispielhaft näher beschrieben. Nur die, die Verschwenkanschläge aufweisen, sind Ausführungsbeispiele gemäß der Erfindung.
Es zeigen:
- Fig. 1: eine perspektivische Ansicht eines ersten Ausführungsbeispiels eines Klemmelementes,
- Fig. 2: eine Seitenansicht des Klemmelementes nach Fig. 1,
- Fig. 3: eine Ansicht des Klemmelementes nach Fig. 1 von hinten,
- Fig. 4: eine weitere perspektivische Ansicht des Klemmelementes nach Fig. 1,
- Fig. 5: eine perspektivische Ansicht eines zweiten Ausführungsbeispiels eines Klemmelementes,
- Fig. 6: eine Seitenansicht des Klemmelementes nach Fig. 5,
- Fig. 7: eine Ansicht des Klemmelementes nach Fig. 5 von oben,
- Fig. 8: eine weitere perspektivische Ansicht des Klemmelementes nach Fig. 5,
- Fig. 9: eine Draufsicht auf eine Verdrehsicherung für ein Gelenkelement nach Fig. 11,
- Fig. 10: eine perspektivische Ansicht der Verdrehsicherung nach Fig. 9 von unten,
- Fig. 11: eine perspektivische Explosionsdarstellung eines Gelenkelementes gemäss einem ersten Ausführungsbeispiel,
- Fig. 12: eine perspektivische Darstellung des Gelenkelementes nach Fig. 11 im blockierten Zustand mit zwei festgeklemmten stabförmigen Elementen,
- Fig. 13: eine Seitenansicht eines Gelenkelementes gemäss einem zweiten Ausführungsbeispiel im blockierten Zustand mit zwei festgeklemmten stabförmigen Elementen,
- Fig. 14: eine Draufsicht auf das Gelenkelement nach Fig. 13,
- Fig. 15: eine teilweise geschnittene Seitenansicht eines Teiles einer Verriegelungsschraube, einer Mutter und einer Sicherungsschraube für ein Gelenkelement nach einer der Fig. 11 bis 14,
- Fig. 16: eine perspektivische Ansicht eines dritten Ausführungsbeispiels eines Klemmelementes gemäss der Erfindung,
- Fig. 17: eine Draufsicht des Klemmelementes nach Fig. 16,
- Fig. 18: eine Ansicht des Klemmelementes nach Fig. 16 von hinten,
- Fig. 19: eine weitere perspektivische Ansicht des Klemmelementes nach Fig. 16,
- Fig. 20: eine perspektivische Ansicht eines vierten Ausführungsbeispiels eines Klemmelementes gemäss der Erfindung,
- Fig. 21: eine Draufsicht des Klemmelementes nach Fig. 20,
- Fig. 22: eine Seitenansicht des Klemmelementes nach Fig. 20,
- Fig. 23: eine weitere perspektivische Ansicht des Klemmelementes nach Fig. 20, ,
- Fig. 24: eine perspektivische Explosionsdarstellung eines Gelenkelementes gemäss einem dritten Ausführungsbeispiel der Erfindung,
- Fig. 25: eine perspektivische Darstellung eines Gelenkelementes gemäss einem vierten Ausführungsbeispiel im blockierten Zustand mit zwei festgeklemmten stabförmigen Elementen,
- Fig. 26: eine Draufsicht auf ein Gelenkelement gemäss einem fünften Ausführungsbeispiel,
- Fig. 27: eine Unteransicht eines Klemmelementes gemäss einem fünften Ausführungsbeispiel gemäss der Erfindung,
- Fig. 28: eine Seitenansicht des Klemmelementes nach Fig. 27,
- Fig. 29: eine Draufsicht des Klemmelementes nach Fig. 27,
- Fig. 30: eine geschnittene Seitenansicht des Klemmelementes nach Fig. 27,
- Fig. 31: eine perspektivische Ansicht des Klemmelementes nach Fig. 27,
- Fig. 32: eine perspektivische Ansicht eines Klemmelementes gemäss einem sechsten Ausführungsbeispiel gemäss der Erfindung,
- Fig. 33: eine Seitenansicht des Klemmelementes nach Fig. 32,
- Fig. 34: eine perspektivische Ansicht eines Klemmelementes gemäss einem siebten Ausführungsbeispiel der Erfindung in Abwandlung des Elementes nach Fig. 16,
- Fig. 35: eine Seitenansicht eines Gelenkelementes gemäss einem sechsten Ausführungsbeispiel der Erfindung, das aus zwei Klemmelementen nach Fig. 34 aufgebaut ist,
- Fig. 36: eine perspektivische Ansicht eines Klemmelementes gemäss einem achten Ausführungsbeispiel gemäss der Erfindung für den Einsatz einer weiteren Verdrehsicherung für ein Gelenkelement,
- Fig. 37: eine perspektivische Explosionsdarstellung eines Gelenkelementes gemäss einem siebten Ausführungsbeispiel der Erfindung,
- Fig. 38: eine Seitenansicht eines Klemmelementes gemäss einem neunten Ausführungsbeispiel gemäss der Erfindung, und
- Fig. 39: eine Draufsicht auf das Klemmelement nach Fig. 38.

### Ausführliche Beschreibung der bevorzugten Ausführungsbeispiele

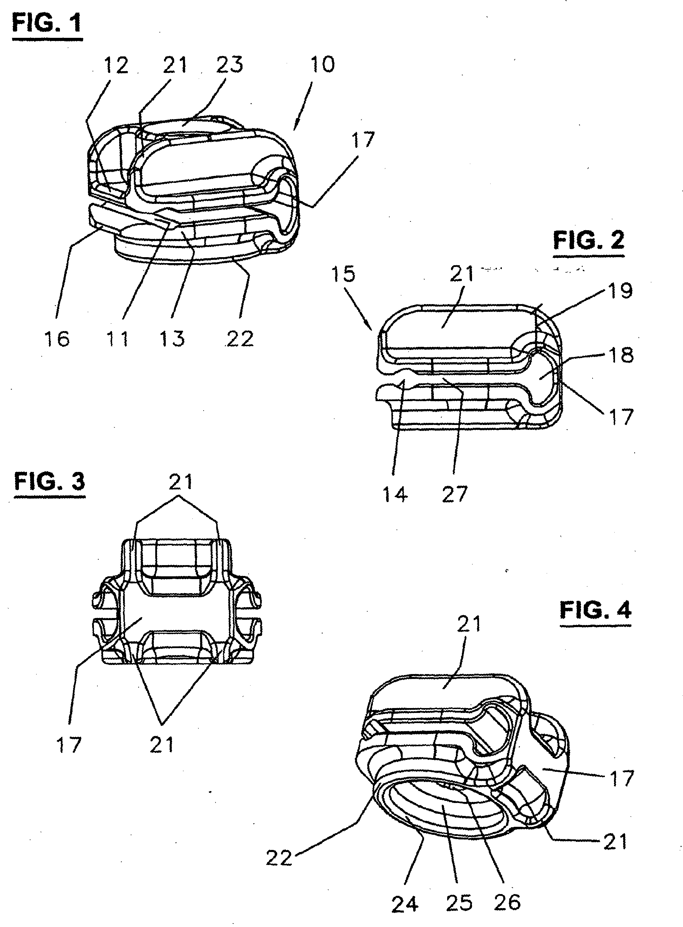

Die Fig. 1 bis 4 zeigen ein ersten Ausführungsbeispiels eines Klemmelementes 10. Die Fig. 1 zeigt dabei eine perspektivische Ansicht, schräg von oben. Das Klemmelement 10 verfügt über zwei gegenüberliegende und einen Hohlraum 11 zur Aufnahme eines stabförmigen Elementes aufweisende Klemmbacken 12 und 13. Die Klemmbacken 12 und 13 verfügen an ihren freien Enden 15 jeweils über eine quer verlaufende Nut 14, die zusammen den Hohlraum 11 aufspannen. An den freien Enden 15 sind die Aussenkanten 16 der aufeinander zugewandten Seite der Klemmbacken 12 und 13 angeschrägt, um das Einschieben eines stabförmigen Elementes von der Seite her zu vereinfachen. Gegenüber dem Hohlraum 11 und den freien Enden 15 ist ein Scharniermittel 17 vorgesehen, welches die Klemmbacken 12 und 13 einstückig miteinander verbindet. Wenn das Klemmelement 10 für einen Stab mit 4 bis 6 Millimetern Durchmesser vorgesehen ist, dann hat die Öffnung an den freien Enden im Ruhezustand einen Durchmesser von beispielsweise 2 Millimetern.

Das Scharniermittel 17 ist bei einer Ausbildung des Klemmelementes 10 aus Kunststoff ein verdünnter Materialbereich, an den sich eine quer und damit parallel zum Hohlraum 11 verlaufende Ausnehmung 18 anschliesst, die auf der dem Scharniermittel 17 zugewandten Seite einen grossen Krümmungsradius aufweist oder mit anderen Worten, dass der Materialquerschnitt des Scharniermittels 17 nur langsam zunimmt. Dies ist insbesondere in der Fig. 2 gut zu erkennen.

Die Fig. 2 zeigt eine Seitenansicht des Klemmelementes 10 nach Fig. 1. Wird der Materialquerschnitt in Bezug auf die radiale Richtung der Krümmung gemessen, so verdoppelt er sich bis zu einem Winkel von 45 Grad. Bis zum Winkel von 90 Grad, also in Richtung zu den Aussenseiten der Klemmbacken 11 und 12, verdreifacht bzw. versechsfacht sich der Materialquerschnitt. Letzterer Materialquerschnitt ist zur Verdeutlichung mit dem Bezugszeichen 19 versehen worden. Zwischen den beiden Klemmbacken 11 und 12 besteht ein Schlitz 27, der beispielsweise in der Ruhelage des Klemmelementes 10 eine konstante Breite aufweist. An beiden Enden geht der Schlitz 27 in die Verbreiterung der Nuten 14 beziehungsweise in die Ausnehmung 18 über.

Aus der Fig. 3 ist eine Ansicht des Klemmelementes 10 von hinten zu erkennen, das heisst auf das Scharniermittel 17. Dieses ist schmaler als die Breite der Klemmbacken 11 und 12 im Bereich der freien Enden 15. Der volle Materialquerschnitt 19, wie er oben angegeben worden ist, besteht nur im Bereich der Querrippen 21, die insbesondere im in den Figuren oberen Bereich der Klemmbacke 12 ausgeprägt sind. Der Bereich zwischen den Querrippen 21 ist dort bis auf eine in der Draufsicht runde Schraubenaufnahme 23 ausgenommen. Die Schraubenaufnahme 23 verfügt beispielsweise über eine konische Schulterfläche oder über eine Stufenschulter, deren Zweck später beschrieben werden wird, und die, was in den Fig. nicht zu sehen ist, in eine durchgehende Bohrung in der oberen Klemmbacke 12 übergeht.

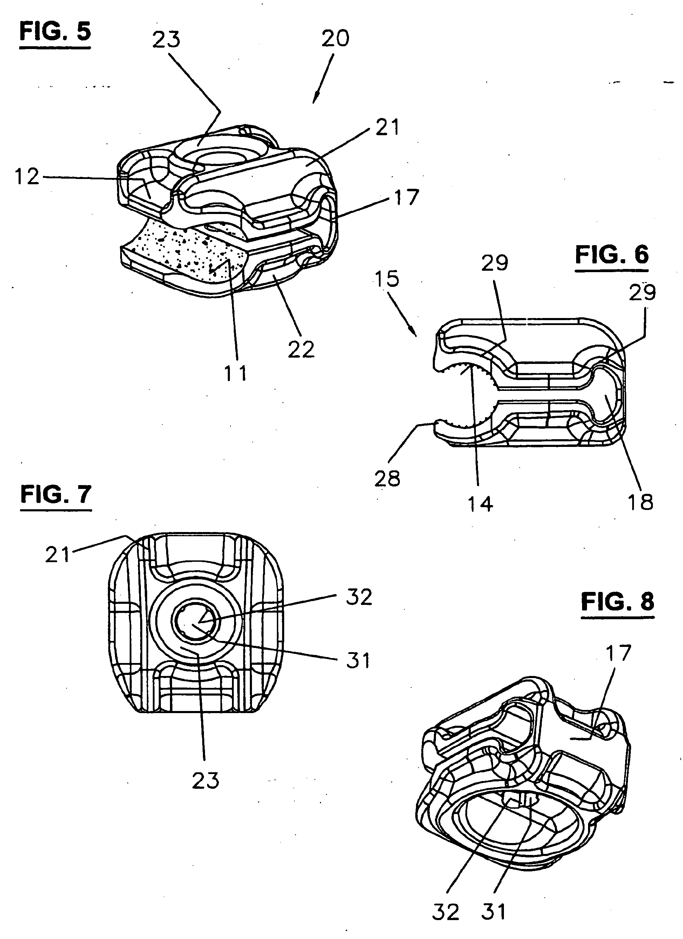

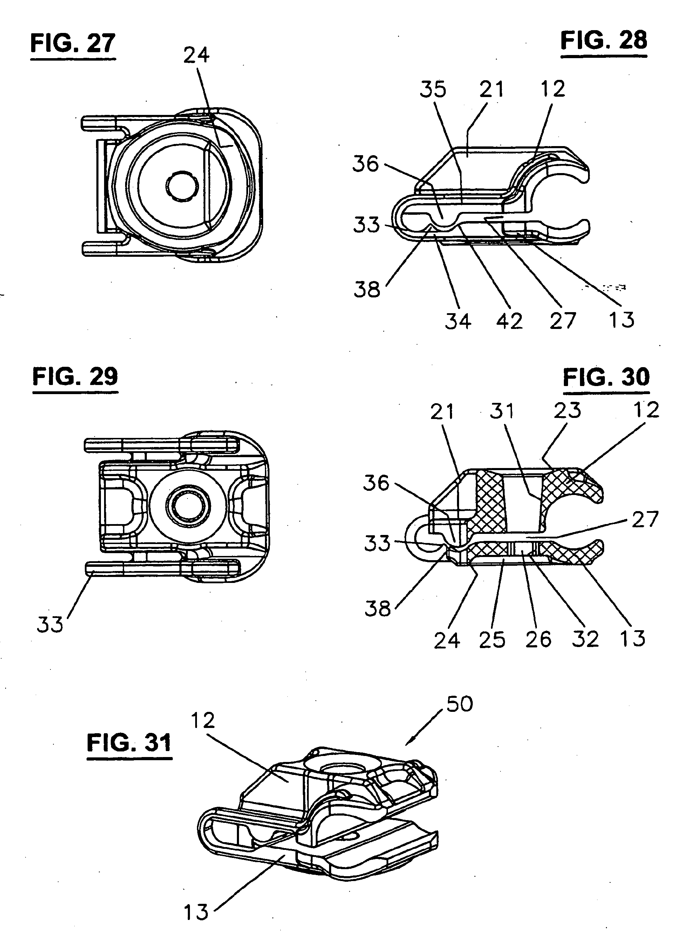

Eine weitere perspektivische Ansicht des Klemmelementes 10 nach Fig. 1 ist in der Fig. 4 dargestellt, diesmal schräg von unten. In der unteren Klemmbacke 13 münden die besagten Querrippen 21 in einem Ringflansch 22, der beispielsweise über einen flachen vertieften ringförmigen Absatz 24 verfügen kann, an den sich eine gewichts-, materialsparende und für die Spritzgussherstellung vorteilhafte Ausnehmung 25 anschliessen kann, in deren Mitte eine Bohrung 26 vorgesehen ist. Diese durchgehende Bohrung 26 ist fluchtend mit der oben genannten durchgehenden Bohrung (Bezugszeichen 31 in der Fig. 7) in der oberen Klemmbacke 12 ausgerichtet. Sie verläuft beim Klemmelement 10 senkrecht zur Achse des Hohlraums 11 und parallel zur Rückseite des Scharniermittels 17. Sie könnte aber auch schräg verlaufen. Die Bohrung 26 ist zylindrisch und sie verfügt an ihrer Innenseite über vier in regelmässigem Abstand angeordnete Führungsrippen 32, die in Fig. 7 und Fig. 30 dargestellt sind. Es können natürlich auch beispielsweise drei oder fünf Rillen 32 vorgesehen sein.

Die Fig. 5 bis 8 zeigen ein zweites Ausführungsbeispiels eines Klemmelementes 20. Gleiche Merkmale sind jeweils mit gleichen Bezugszeichen versehen. Die Fig. 5 zeigt dabei eine perspektivische Ansicht, schräg von oben. Der einzige Unterschied zwischen den beiden Klemmelementen 10 und 20 nach Fig. 1 und Fig. 5 liegt darin, dass das Klemmelement 20 zur Aufnahme eines im Durchmesser grösseren stabförmigen Elementes vorgesehen ist. Dies bedeutet, dass die Nuten 14 grösser ausgestaltet sind. Die Mittelachse des Hohlraums 11 liegt aber immer noch in der Höhe des Schlitzes 27. Die Nuten 14 sind hier so gross ausgestaltet, dass die freien Enden 15 nach aussen nur noch einen abgerundeten Bereich 28 bilden, der ein seitliches Einführen von Stäben vereinfacht. Wie Kotflügel 29 wird die Nut 14 beziehungsweise die Ausnehmung 18 überspannt, um die Steifigkeit des Klemmelementes 20 (oder auch 10) zu gewährleisten. Wenn das Klemmelement 20 für einen Stab mit 12 Millimetern Durchmesser vorgesehen ist, dann hat die Öffnung an den freien Enden im Ruhezustand einen Durchmesser von beispielsweise 9 Millimetern.

Die Fig. 6 und 8 zeigen wiederum das Klemmelement 20 in einer Seitenansicht beziehungsweise in einer perspektivischen Ansicht schräg von unten.

Fig. 7 zeigt eine Ansicht des Klemmelementes 20 von oben. Angrenzend an die konische Schulter 23 folgt die zentrale durchgehende Bohrung 31. Die hier erkennbaren Führungsrippen 32 gehören zu der Innenwand der Bohrung 26. Die Bohrung 31 kann auch zylindrisch sein, sie ist aber vorzugsweise konisch, wie dies in der Fig. 30 dargestellt ist.

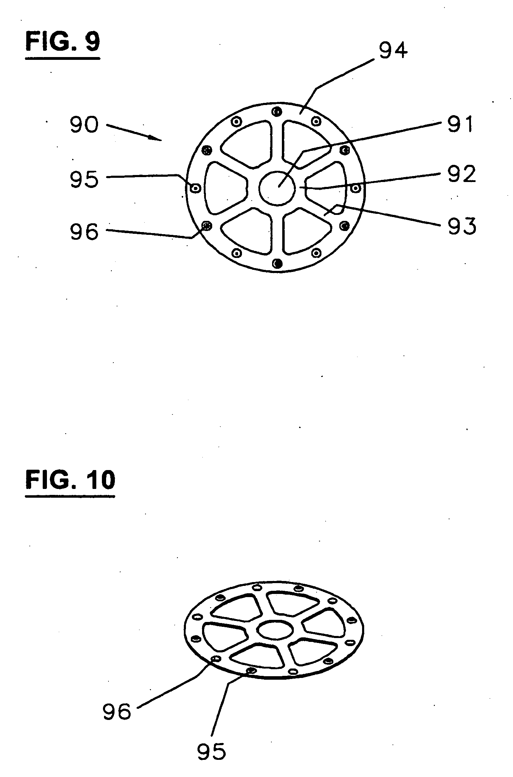

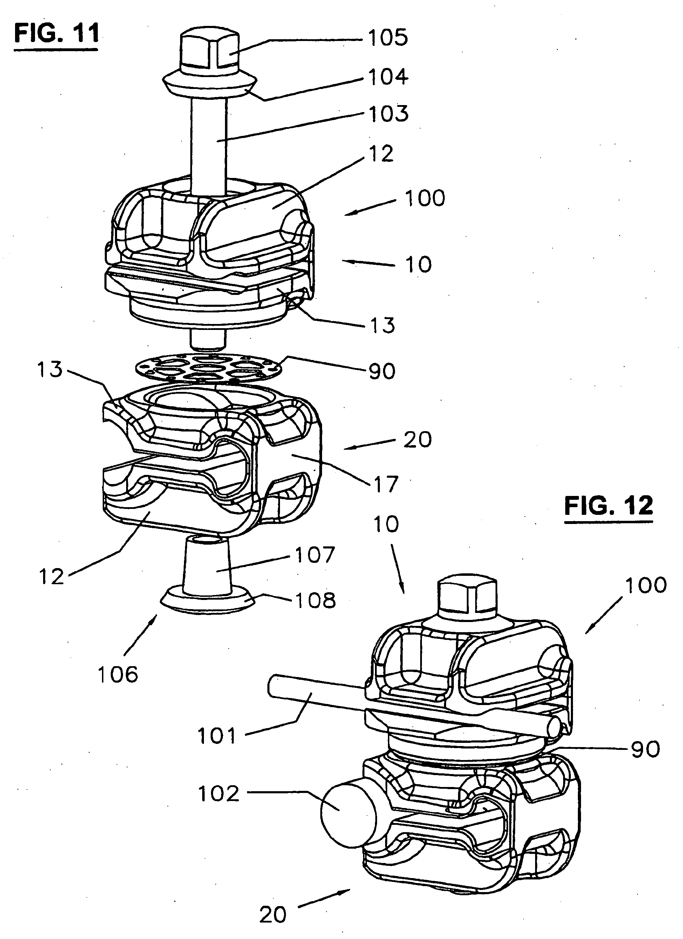

Die Fig. 9 zeigt eine Draufsicht auf eine Verdrehsicherung 90 für ein Gelenkelement nach Fig. 11. Die Verdrehsicherung 90 ist beispielsweise ein dünnes Metallplättchen, das über eine zentrale Bohrung 91, eine Nabe 92 und Speichen 93 verfügt. Am äusseren Kranz 94 sind beispielsweise aufeinander folgend punktförmige Erhöhungen 95 und Vertiefungen 96 vorgesehen. Diese sind beispielsweise so angeordnet, dass die Vertiefungen 96 immer gegenüber den hier sechs Speichen 93 angeordnet sind, wobei Erhöhungen 95 jeweils intermittierend vorgesehen sind.

Die Fig. 10 zeigt diese Verdrehsicherung 90 nun schräg von unten. Es ist zu erkennen, dass hier die Erhöhungen 95 im Bereich der Speichen 93 sind. Dies gestattet den Einsatz eines einfachen Stanzverfahrens zur Herstellung der Plättchen der Verdrehsicherung 90. Die punktförmige Erhöhungen 95 und Vertiefungen 96 können rund sein, pyramidal, oder polygonförmig. Sie können in mehreren Reihen radial nebeneinander verlaufen, in grösserer Anzahl als in der Fig. 9 etc.. Bei einem anderen Ausführungsbeispiel können auch radiale Rippen vorgesehen sein. Sofern das Klemmelement 10, 20 keine Ausnehmung 25 aufweist, sondern nur die Stufe 24, kann die Verdrehsicherung 90 auch ein Vollmaterialplättchen mit entsprechenden Erhebungen/Vertiefungen sein. Wesentlich ist die sich aus der nachfolgenden Beschreibung eines vorteilhaften Gelenkelementes 100 ergebende Funktion der Verdrehsicherung 90. Eine solche Verdrehsicherung 90 kann auch durch Gestaltung des Materials der ersten Klemmbacke 13 erreicht werden.

Die Fig. 11 zeigt eine perspektivische Explosionsdarstellung eines Gelenkelementes 100 gemäss einem ersten Ausführungsbeispiel. Fig. 12 zeigt das Gelenkelement 100 nach Fig. 11 im blockierten Zustand mit zwei festgeklemmten stabförmigen Elementen 101 und 102. Das stabförmige Element 101 ist ein dünner Steckverbinder und das stabförmige Element 102 ist ein dicker Befestigungsstab. Das Gelenkelement 100 besteht aus fünf Teilen. Dies sind zwei identische Klemmelemente 10 oder 20 oder jeweils ein Klemmelement 10 für ein dünnes stabförmiges Element 101 und ein Klemmelement 20 für ein dickes stabförmiges Element 102. Diese sind mit ihren den Flansch 22 aufweisenden Seiten aufeinander zugerichtet positioniert. Zwischen diesen beiden Flanschen 22 ist die Verdrehsicherung 90 angeordnet.

Durch die dann fluchtenden Bohrungen 31, 91 und 26 ist eine Schraube 103 eingesetzt, die sich mit ihrem konischen Flansch 104 auf der konischen Schraubenaufnahme 23 absetzen kann. Die Schraube 103 verfügt zur Betätigung beispielsweise über einen Vierkant 105. Es ist klar, dass statt einem Vierkant auch ein Sechskant oder ein Schlitz etc. vorgesehen sein kann. Die Schulter 104 ist vorzugsweise komplementär zur Aufnahme 23 ausgestaltet. Von der anderen Seite her wird eine Mutter 106 aufgesetzt. Die Mutter 106 verfügt über eine leicht konische Hülse 107 und einen konischen Flansch 108 als Abdeckhaube. Die Form des Flansches 108 entspricht der Form der Schraubenaufnahme 23 des Klemmelementes 10 oder 20. Die Hülse 107 wird in die Bohrung 31 eingesetzt und ragt vorteilhafterweise bis in die Bohrung 26 und/oder durch diese hindurch. Die Hülse 107 wird im Presssitz eingepasst, sie kann zusätzlich auch ein Aussengewinde aufweisen. Dieses kann passend zu einem in der Bohrung 26 vorgesehenen Innengewinde ausgestaltet sein.

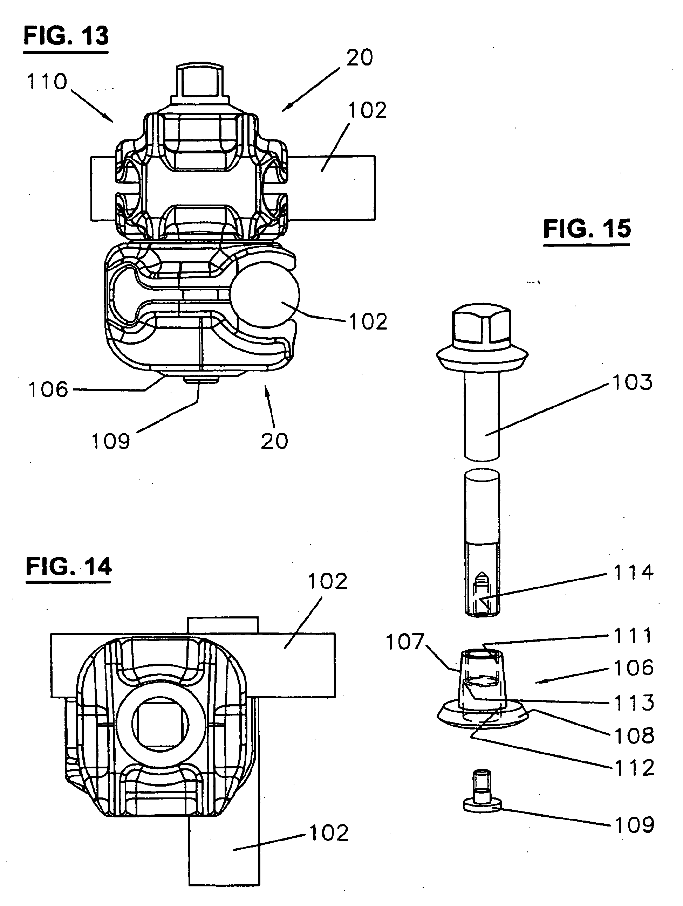

Bei einer anderen, in den Zeichnungen nicht dargestellten Ausführungsform ist ein Klemmelement mit einer kippbaren aber verdrehsicheren Lagerung für die Mutter versehen. Die Klemmbacke 12 verfügt wieder über die konisch sich öffnende Bohrung 31. Diese Bohrung 31 weist aber, vorteilhafterweise auf der von dem Hohlraum 11 wegweisenden Seite über eine Ausnehmung, die insbesondere ein rechteckiger Schlitz sein kann. Bei der Montage wird in die Ausnehmung die Mutter eingesetzt, die zylindrisch ist. Ansonsten kann die Befestigung wie in der Fig. 15 dargestellt ausgeführt sein. Durch die zylindrische Mutter besteht ein Spiel, so dass beim Einklipsen eines Stabes 102 in den Hohlraum 11 auch der Oberteil 12 des Klemmelementes verkippt werden kann. Um die Fixierung der Schraube 103 zu gewährleisten und die Mutter verdrehsicher zu gestalten, weist diese einen Ansatz oder Nase auf, der mit seitlichem Spiel in die besagte Ausnehmung ragt. In einer Seitenansicht des Klemmelementes hat die Nase Spiel in der Ausnehmung, um die besagte Kippbewegung des Oberteils 12 zu gestatten. Neben der Mutter mit Nase sind auch andere Ausgestaltungen möglich, beispielsweise eine L-förmig abgeplattete Mutter, die beispielsweise taumelgenietet und gestanzt ist, so dass ein Fortsatz in eine entsprechende Nut in dem Oberteil 12 ragt und die Verdrehsicherung bewirkt.

Die Mutter 106 verfügt über ein Innengewinde, welches zu dem komplementären Aussengewinde der Schraube 103 passt.

Die Fig. 12 zeigt dann das zusammengebaute Gelenkelement 100 mit einem Klemmelement 10 für einen dünnen Stab 101 und ein Klemmelement 20 für einen dickeren Stab 102. Durch das Anziehen der Schraube 103 gegenüber der Mutter 106 werden die beiden Klemmelemente 10 und 20 aneinandergezogen, da die durch die Scharniermittel 17 ausgeübten Kräfte die beiden unteren Klemmbacken 13 der Klemmelemente 10 bzw. 20 aufeinander zu bewegen.

Dann kann durch Ausüben von Druck seitlich ein Stab 101 oder 102 in den jeweiligen Hohlraum eingeführt werden. Da der Durchmesser des Stabes 101 oder 102 grösser als die Öffnung an den freien Enden 15 ist, ist er gegen Herausfallen gesichert. Bei einer in den Zeichnungen nicht dargestellten Aufrauhung der Nuten 14 ist er auch gegen eine einfache longitudinale Verschiebung gesichert.

Ferner üben die beiden Scharniermittel 17 einen Druck auf die Verdrehsicherung 90 aus, so dass die Klemmelemente 10 bzw. 20 selbst gegen ein leichtes Verdrehen gegeneinander um die Achse der Schraube 103 geschützt sind. Die Erhebungen 95 drücken sich jeweils, das heisst nach der Beschreibung der Fig. 9 und 10 in beiden Richtungen senkrecht zur Plättchenebene, in den eine Stufe bildenden ringförmigen Absatz 24. Damit sind jeweils die Klemmbacken 13 und damit die Klemmelemente 10 bzw. 20 gegen ein leichtes Verdrehen gesichert.

Bei einem weiteren Anziehen der Schraube 103 werden die Klemmbacken 12 und 13 gegen die rückstellende Kraft der Scharniermittel 17 weiter aufeinander zu bewegt und schliesslich durch das dazwischen gelegte Plättchen der Verdrehsicherung 90 in ihrer Winkelposition vollständig blockiert. Damit werden gleichzeitig die Stäbe 101 und 102 in den Nuten 14 durch Verkleinern des Hohlraumes 11 gegen longitudinales Verschieben als auch gegen Verdrehen vollständig gesichert. Eine Sicherungsschraube 109 wird erst im Zusammenhang mit der Fig. 13 dargestellt, kann aber auch hier Anwendung finden. Die Mutter 106 ist als durchgehende Hülse ausgebildet.

Bei einem Öffnen der Schraube 103 verbleibt die Mutter 106 in dem einen Klemmelement. Die Verdrehsicherung 90 hat sich in das weichere Material der Stufe 24 eingedrückt. Dies spricht - ausser einem sofortigen Anziehen der Schraube an diesem oder einem anderen Ort eines Fixateur externe dieses Patienten für ein Wegwerfen des so gebildeten Gelenkelementes 100. Die Einpressspuren der Verdrehsicherung in die Stufe 24 ist ein Gebrauchszeichen für das Klemmelement, so dass der Benutzer erkennen kann, dass die Wiederverwendung des Produktes auszuschliessen ist.

Die Fig. 13 zeigt eine Seitenansicht eines Gelenkelementes 110 gemäss einem zweiten Ausführungsbeispiel im blockierten Zustand mit zwei festgeklemmten dicken stabförmigen Elementen 102. Daher sind bei diesem Ausführungsbeispiel zwei Klemmelemente 20 nach Fig. 5 eingesetzt.

Die Fig. 14 zeigt eine Draufsicht auf das Gelenkelement nach Fig. 13, bei der die beiden Stäbe 102 um 90 Grad zueinander verdreht angeordnet sind.

Die Fig. 15 zeigt eine teilweise geschnittene Seitenansicht einer Verriegelungsschraube 103, einer Mutter 106 und einer Sicherungsschraube 109 für ein Gelenkelement 100, 110 nach einer der Fig. 11 bis 14. Der Konus 107 der Mutter 106 kann glatt sein, er kann aber auch ein Gewinde aufweisen. Dann ist vorzugsweise auch die Bohrung 31 mit einem Gewinde versehen. Die Hülse der Mutter 106 verfügt über eine durchgehende Bohrung mit einem ersten schmaleren Abschnitt 111, der ein Innengewinde zur Aufnahme des Aussengewindes der Schraube 103 aufweist. Ein daran anschliessender breiter zweiter Abschnitt 112 verfügt über eine Schulter 113, auf der sich der Schraubenkopf der Sicherungsschraube 109 absetzen kann. Die Sicherungsschraube 109 ist dafür vorgesehen, sich in ein Innengewinde 114 in der Spitze der Schraube 103 einzugschrauben. Dabei kann insbesondere vorgesehen sein, beispielsweise durch Klebstoff oder ein anderes Mittel, dass nach einem Vorzusammenbau eines Gelenkelementes 100 oder 110 sich die Verriegelungsschraube 109 nicht mehr aus dem Gewinde 114 lösen lässt, womit die Einheit des Gelenkelementes 100 oder 110 gesichert ist.

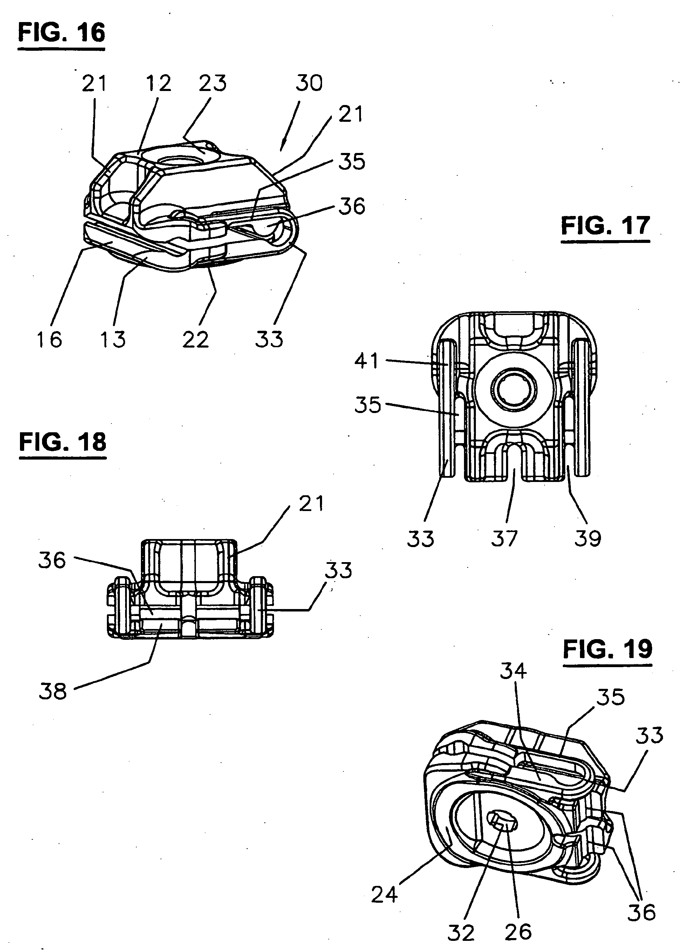

Die Fig. 16 bis 19 zeigen ein drittes Ausführungsbeispiel eines Klemmelementes 30 gemäss der Erfindung in einer perspektivischen Ansicht, einer Draufsicht, einer Rückansicht und einer weiteren perspektivischen Ansicht. Der Unterschied zwischen den ersten zwei Ausführungsbeispielen und den nun folgenden liegt darin, dass das Scharniermittel 17 bei den ersten beiden Ausführungsbeispielen direkt mit dem Körper der Klemmbacken 12 beziehungsweise 13 verbunden ist und direkt in die Rippen 21 übergeht. Die Klemmbacken 12 beziehungsweise 13 sind an der gesamten Rückseite des Klemmelementes 10 oder 20 miteinander verbunden.

Bei dem Klemmelement 30 sind die beiden Klemmbacken 12 und 13 nur über zwei seitliche Verbindungsstege 33 verbunden. Die Verbindungsstege 33 umfassen jeweils einen unteren kurzen Übergangssteg 34 in die untere Klemmbacke 13 und einen langen Steg 35, der parallel zu der oberen Klemmbacke 12 verläuft, bevorer im Bereich der Wandung 41 des Hohlraums 11 in die obere Klemmbacke 12 übergeht. Zwischen jedem der beiden Verbindungsstege 33 und der oberen Klemmbacke 12 ist ein Trennschlitz 39, so dass die beiden einander gegenüberliegenden Elemente 36 und 38 von oberer bzw. unterer Klemmbacke 12 bzw. 13 getrennt sind. An der oberen Klemmbacke 12 ist ein Halbzylinder 36 vorgesehen, dem in einem Abstand, der beispielsweise die Hälfte oder ein Drittel der Höhe des Schlitzes 27 entspricht, eine komplementäre Rille 38 gegenüberliegt. Halbzylinder 36 und komplementäre Rille 38 sind in der Mitte durch einen Schlitz 37 in jeweils zwei voneinander getrennte Abschnitte unterteilt. Die dargestellten Ausführungsbeispiele können auch ohne Schlitz 37 ausgeführt sein, so dass ein Halbzylinder 36 und eine Rille 38 gegenüberliegen. Dann ist das Lager nicht in der Mitte unterteilt. Es ist nicht notwendig, dass ein Klemmelement nach dem dritten oder vierten Ausführungsbeispiel eine Aufnahme für eine Verdrehsicherung aufweist.

Die anderen Merkmale der unteren Klemmbacke 13, das heisst die Aufnahme 24 für eine Verdrehsicherung 90 und die Bohrung 26 mit den Führungsrippen 32, und der oberen Klemmbacke 12, das heisst die Aufnahme 23, die konische Bohrung 31 und die Rippen 21 können gleich ausgestaltet wie bei den ersten beiden und dem letzten Ausführungsbeispiel. Bei den Rippen 21 besteht ein Unterschied darin, dass sie bei den Klemmelementen 10 und 20 eine direkte Verlängerung des Scharniermittels 17 sind. Bei den Klemmelementen 30 und 40 sind die Scharniermittel im Ruhezustand durch den Verbindungssteg 33 gebildet, der nur indirekt über die Wandung 41 des Hohlraums 11 mit den Rippen 21 verbunden ist.

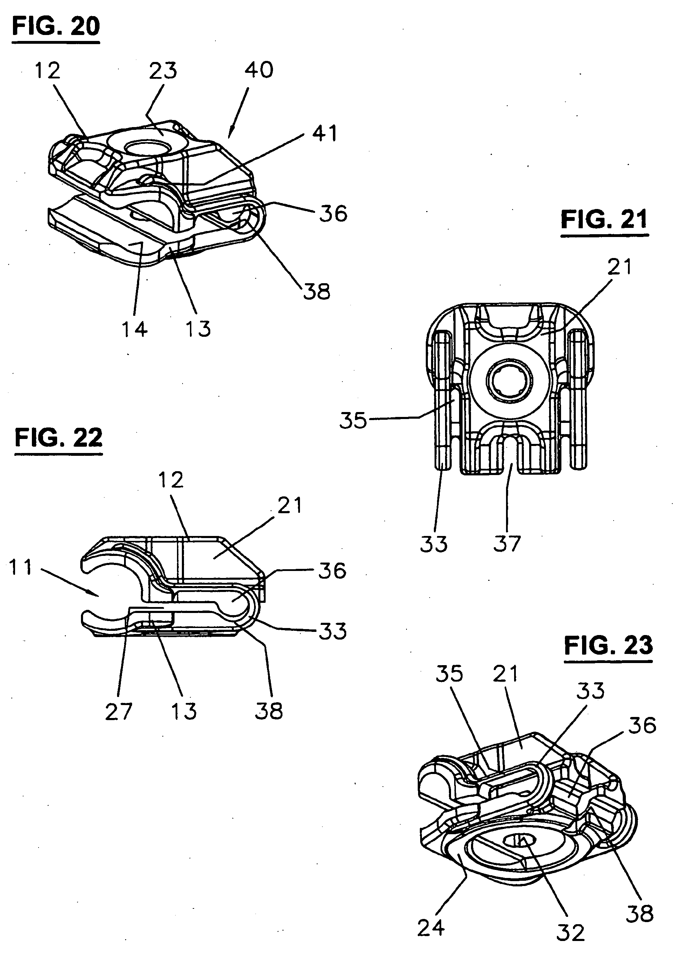

Die Fig. 20 bis 23 zeigen ein viertes Ausführungsbeispiel eines Klemmelementes 40 gemäss der Erfindung, in einer perspektivischen Ansicht, in einer Draufsicht, in einer Seitenansicht und in einer weiteren perspektivischen Ansicht. Hier ist der Hohlraum 11 gross ausgestaltet, um ein dickes stabförmiges Element 102 aufzunehmen. In den Fig. ist gut zu erkennen, wie der lange obere Steg 35 in die Wandung 41 übergeht und dabei fast in einem Viertelkreis als Rippe auf der besagten Wand 41, räumlich getrennt von der Rippe 21 bestehen bleibt.

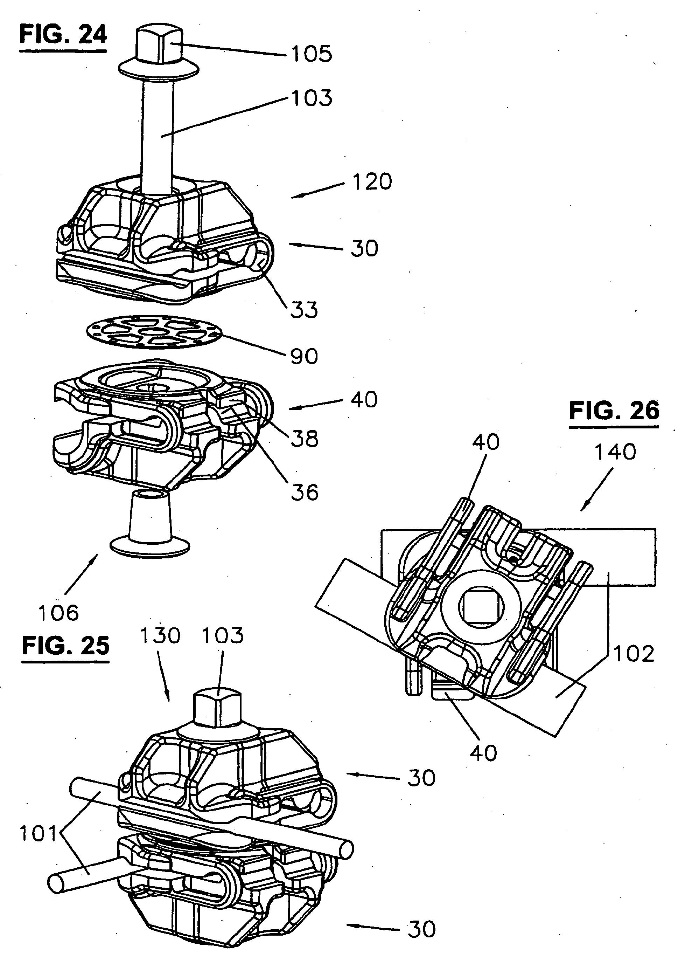

Die Fig. 24 zeigt eine perspektivische Explosionsdarstellung eines Gelenkelementes 120 gemäss einem dritten Ausführungsbeispiel der Erfindung, Fig. 25 eine perspektivische Darstellung eines Gelenkelementes 130 gemäss einem vierten Ausführungsbeispiel im blockierten Zustand mit zwei festgeklemmten dünnen stabförmigen Elementen 101 und Fig. 26 eine Draufsicht auf ein Gelenkelement 140 gemäss einem fünften Ausführungsbeispiel. Der Aufbau dieser Gelenkelemente 120 und 130 entspricht dem der ersten beiden Ausführungsbeispiele von Gelenkelementen 100 und 110. Sofern zwei gleichstarke stabförmige Elemente 101 bzw. 102 zu klemmen sind, werden die beiden gleichen Klemmelemente 30 bzw. 40 eingesetzt, wobei die unteren Klemmbacken 13 einander gegenüberliegend Zwischen diesen ist ebenfalls wieder eine Verdrehsicherung 90 vorgesehen. Für unterschiedliche Klemmstärken werden jeweils ein Klemmelement 30 und ein Klemmelement 40 eingesetzt.

Im Ruhezustand der Klemmelemente 30 und 40 werden die Klemmbacken 12 und 13 im Abstand des Schlitzes 27 durch die Federkraft der beiden seitlichen Stege 33 gehalten. Bei einem Anziehen der Schraube 103 wird der Schlitz verkleinert. Durch die zentrale Krafteinleitung über die Schrauben- und Mutterelemente 104 und 108 auf die identischen Flächen 23 wird der Schlitz 27 in seiner Dicke verkleinert, bis die Nut 14 den im Hohlraum 11 befindlichen Stab 101 oder 102 kontaktieren. Dann verschwenkt die die Rippen 21 aufweisende (obere) Klemmbacke 12 um den Stab 101 oder 102 und der Halbzylinder 36 setzt auf der komplementären Fläche 38 auf. Bei einem weiteren Anziehen der Schraube 103 beginnt ab diesem Zeitpunkt die Blockierwirkung und die Einheit Halbzylinder 36 - komplementäre Fläche 38 übernimmt die Scharnierfunktion vom Steg 33 und vermeidet eine zu hohe Belastung derselben.

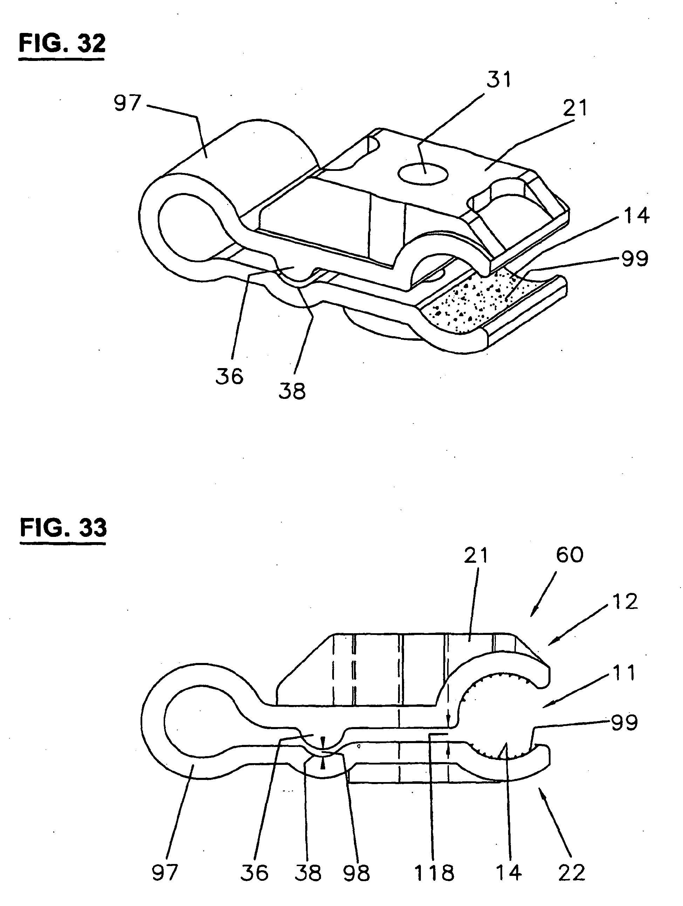

Dies kann auch beispielsweise wie in den Fig. 32 und 33 gelöst werden, die später beschrieben werden.

Die Fig. 27 bis 31 zeigen ein Klemmelement 50 gemäss einem fünften Ausführungsbeispiel gemäss der Erfindung, in einer Unteransicht, einer Seitenansicht, einer Draufsicht, einer geschnittenen Seitenansicht und einer perspektivischen Ansicht. Dieses Klemmelement 50 ist für ein dickes stabförmiges Element 102 vorgesehen. Natürlich kann es auch so abgewandelt werden, um ein dünnes stabförmiges Element 102 aufzunehmen. Ein wesentlicher Unterschied zwischen dem Klemmelement 40 (oder 30) und diesem liegt darin, dass die oberen langen Stege 35 direkt vom Körper der Klemmbacke 12 abgehen und damit den Aussenrand des Klemmelementes 50 begrenzen. Die Stege 35 sind insbesondere auch nicht als Rippen auf der Wandung 41 des Hohlraums 11 ausgebildet. Die Halbzylinder 36 und komplementäre Rille 38 sind nicht durch einen Schlitz 37 aufgetrennt. Die halbkreisförmigen Übergänge der langen Stege 35 und kurzen Übergangsstege 34 in die Halbzylinder 36 und komplementäre Rille 38 sind ausladender und stehen über diese um den gesamten Radius des Überganges hinaus. Der kurze Übergangssteg 34 umfasst hier eine Übergangsstufe 42, die fast der Materialdicke des Steges 33 entspricht. Der Steg 33 ist hier wie in den anderen Ausführungsbeispielen beispielsweise zwischen 1 und 5, vorzugsweise zwischen 2 und 3 Millimeter dick. Die in dem Querschnitt der Fig. 30 gut zu erkennende Ausgestaltung der Bohrungen 26 und 31 kann die folgenden Masse haben. Eine zylindrische Bohrung 26 in der unteren Klemmbacke 13 hat einen Durchmesser von 6,5 Millimeter, wobei die Rippen 32 den Durchmesser auf 5,8 Millimeter verkleinern. Die konische Bohrung 31 hat beispielsweise einen Winkel zwischen 5 und 10, hier 8 Grad und geht in der Tiefe von einem Durchmesser von 8,5 Millimeter aus. Der Winkel der konischen Schraubenaufnahme 23 ist beispielsweise zwischen 45 und 75, hier 60 Grad, die Höhe beispielsweise 2 Millimeter.

Die Fig. 32 und 33 zeigen eine perspektivische Ansicht eines Klemmelementes 60 gemäss einem sechsten Ausführungsbeispiel gemäss der Erfindung, beziehungsweise eine Seitenansicht dieses Klemmelementes 60. Dieses Ausführungsbeispiel zeigt zudem die Anordnung von Rutschsicherungselementen 99. Diese sind hier regelmässig verteilte pyramidale Erhöhungen auf der Oberfläche der Nut 14. Diese Erhöhungen 99 können auch noppenförmig ausgestaltet sein. Wesentlich ist, dass sie insbesondere die dickeren stabförmigen Elemente 102 vor einem Verdrehen oder longitudinalen Verrutschen in der Klemme sichern.

Das sechste Ausführungsbeispiel nach Fig. 32 und 33 verfügt über die Halbzylinder 36 und Rille 38. Wie schon erwähnt, können diese Elemente auch flach einander gegenüberliegen. Wesentlich ist, dass das Scharnierelement 97 bei seinem Übergang in die Klemmenhälften 12 und 22 einen kleineren Abstand 98 dieser Klemmenhälften 12 und 22 aufweist als in der Nähe des Hohlraums 11. Der dortige Abstand 118 ist erheblich grösser als der Abstand 98. Damit ist gewährleistet, dass beim Anziehen einer Schraube und dem Schliessen der Klemmbacken sich zuerst der Abstand 98 auf Null reduziert, so dass die Last der Verklemmung ab diesem Zeitpunkt von dem Kontaktpunkt oder der Kontaktlinie der komplementären Elemente 36 und 38 übernommen wird und die Verschwenkachse in diese Kontaktlinie verschoben wird. Damit wird die Federwirkung umgesetzt und im Effekt verstärkt.

Das Scharnierelement 97 beim sechsten Ausführungsbeispiel ist ein federnder fast geschlossener Zylinder in einem Abstand hinter der besagten Kontaktlinie. Er öffnet sich insbesondere nach oben, um nicht über die Unterseite 22 des Klemmelementes 60.hinauszuragen.

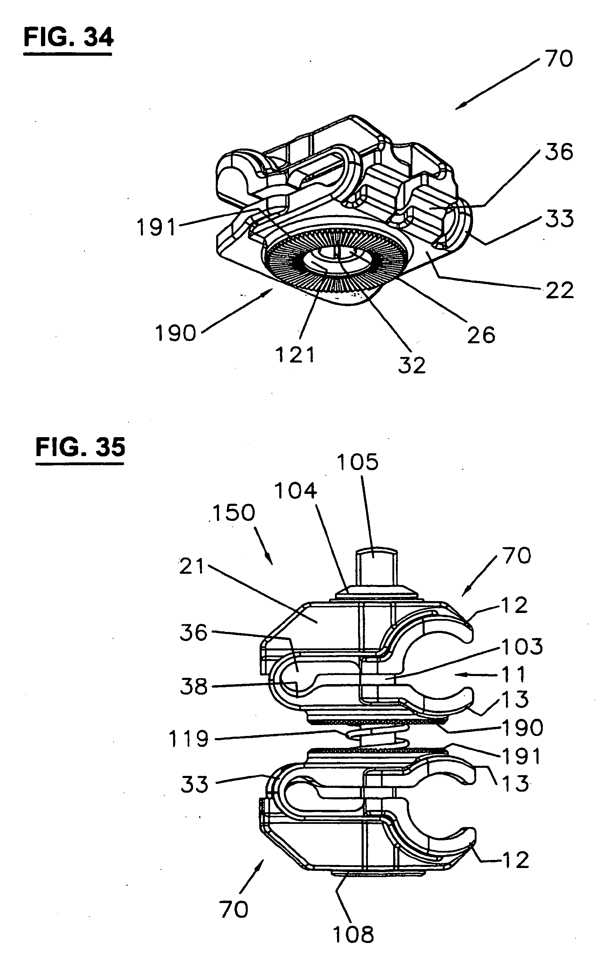

Die Fig. 34 zeigt eine perspektivische Ansicht eines Klemmelementes 70 gemäss einem siebten Ausführungsbeispiel gemäss der Erfindung in Abwandlung des Elementes nach Fig. 16.

Die Fig. 35 zeigt eine Seitenansicht eines Gelenkelementes 150 gemäss einem sechsten Ausführungsbeispiel der Erfindung, das aus zwei Klemmelementen 70 nach Fig. 34 aufgebaut ist. Die Verdrehsicherung 190 ist aus in einer Ebene liegenden radialen Prismen 191 aufgebaut, wobei in der Mitte die durchgehende Bohrung 26 besteht. In einer anderen Ausführungsform können die radialen Prismen 191 auch konisch ausgerichtet sein, so dass bei komplementären Prismen 191 in einem zweiten Klemmelement ein selbstzentrierendes-Gelenkelement 150 geschaffen wird. Das in den Fig. 34 und 35 dargestellte Ausführungsbeispiel hat dagegen den Vorteil, dass die beiden Verdrehsicherungen 190 von identischen Klemmelementen 70 direkt ineinander eingreifen können.

Bei dem Gelenkelement 150 nach Fig. 35 ist zwischen den beiden Klemmelementen 70 eine Spiralfeder 119 angeordnet, die sich in der Federaufnahme 121 abstützt. Die Federaufnahme 121 kann wie in Fig. 34 eine halbkugelige Fläche bilden; sie kann auch eben und glatt sein; sie kann insbesondere auch rauh sein, um eine grösseren Widerstand der Feder 119 gegen Verdrehen zu gewährleisten. Die Feder 119 drückt die beiden Klemmelemente 70 voneinander weg und soll das Verdrehen der beiden Klemmelemente 70 gegeneinander sichern. Sie sichert nicht das Spreizen der Backen 12 und 13; dafür sind die federnden Scharniermittel 17, 33 und 97 vorgesehen, die sich gegen die beim Einklipsen von Stäben 101 und 102 wirkenden Kräfte öffnen. Die Feder 119 kann auch ein Tellerfederpaket oder ein anderes federndes Element sein.

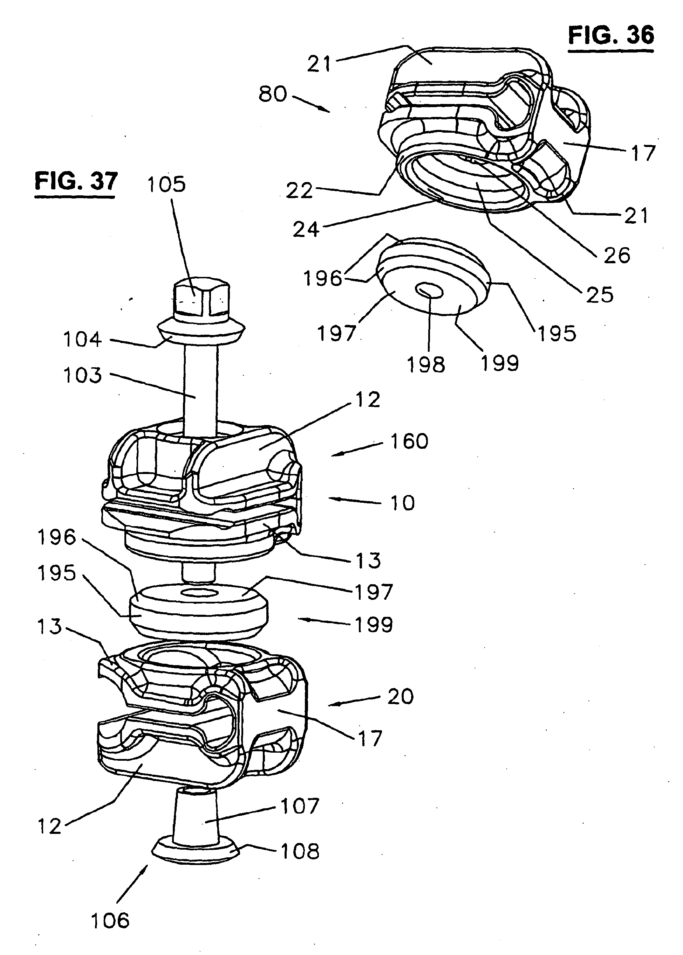

Insbesondere kann es auch ein flexibles Kunststoffschaumelement 199 wie nach Fig. 36 sein. Erst beim Anziehen der Schraube 103 greifen dann die Verdrehsicherungen 190 ineinander und setzen die Winkelstellung des Gelenkelementes 160 wie nach Fig. 37 fest.

Die Fig. 36 zeigt eine perspektivische Ansicht einer weiteren Verdrehsicherung 199 für ein Gelenkelement im Zusammenspiel mit einem dafür ausgestatteten achten Ausführungsbeispiel eines Klemmelementes 80. Dies ist ein flexibles zylindrisches Element 199 mit einer zentralen Bohrung 198 zur Durchführung der Schraube 103. Es kann an Stelle der Verdrehsicherung 90 eingesetzt werden und ebenfalls die Feder 119 ersetzen. Dafür ist sein Material vorteilhafterweise an den Boden- und Deckelflächen 197 härter und kann insbesondere auch strukturiert sein oder harte Einsätze umfassen, um in den ringförmigen Absatz 24 einzugreifen. Das Klemmelement 80 ist hier entsprechend dem Ausführungsbeispiel nach Fig. 1 ausgestaltet, nur die Tiefe und die Seitenwände sind für die Aufnahme der Verdrehsicherung 199 vorgesehen. Im Zylinderbereich ist das Element 199 flexibel, um zusammengedrückt werden zu können, wenn die Schraube 103 angezogen wird. Dabei können sich die Unterseiten des Klemmelementes 80 eines Gelenkelementes 160 nach Fig. 37 berühren. Die Verdrehsicherung ist angefast und verfügt zwischen Mantelfläche 195 und Deckel- bzw. Bodenfläche 197 über konische Abschrägungen 196.

Das Material der Verdrehsicherung 199 ist im Boden- und Deckelbereich vorteilhafterweise härter als das Material der eingesetzten Klemmelemente, und besteht bevorzugterweise im Vollmaterialteil aus einem flexiblen, zusammendrückbaren Material, insbesondere einem Kunststoffschaum.

Die Fig. 37 zeigt eine perspektivische Explosionsdarstellung eines Gelenkelementes 160 gemäss einem siebten Ausführungsbeispiel.

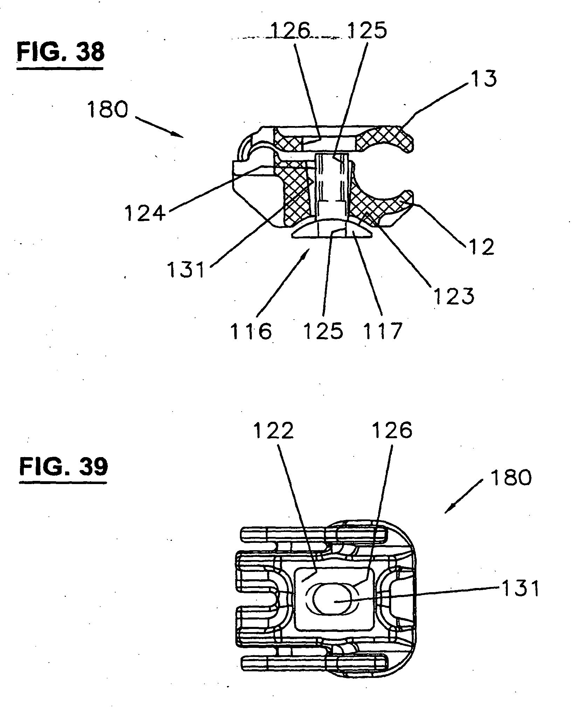

Die Fig. 38 zeigt eine Seitenansicht eines Klemmelementes 180 gemäss einem neunten Ausführungsbeispiel gemäss der Erfindung mit einer zugehörigen Mutter und Fig. 39 eine Draufsicht auf das Klemmelement 180 nach Fig. 38. Die Bohrung 126 in der Klemmbacke 13 ist im Durchmesser grösser als in anderen vorgenannten Ausführungsbeispielen. Die Bohrung 131 in der Klemmbacke 12 ist leicht konisch, wobei der kleinere Durchmesser im Bereich der äusseren Mündung ist, die besser in der Fig. 39 zu erkennen ist. Die Mündung endet in der Mutterkopfaufnahme 122, die halbkugelig konkav ist, wobei der Kugeldurchmesser ungefähr dem unteren Krümmungsradius der Mutternkopfrückseite 123 des Mutterkopfes 117 der Mutter 116 entspricht. Die Mutter 116 hat eine zylindrische Hülse 124, die innen aufgebohrt ist und ein Innengewinde 125 aufweist, um die Befestigungsschraube 103 aufzunehmen. Die Bohrung der Hülse 124 kann mit einem kleineren Durchmesser 125 durchgehend ausgestaltet sein, um die Sicherungsschraube 109 nach Fig. 15 aufzunehmen.

Wesentlich ist hier, dass die Mutter 116 mit ihrem Kopf 117 in der Aufnahme 122 gegenüber dem Klemmelement 180 beweglich ist, in ähnlicher Art und Weise, wie es die Schraube 103 gegenüber seinem Klemmelement ist. Das vereinfacht das Aufklipsen von Stäben 102 oder 102, insbesondere von dicken Stäben 102. Dabei ist die Mutternkopfaufnahme 122 im Bereich der Mündung der Bohrung 131 asymmetrisch ausgestaltet, das heisst die kugelige Ausnehmung ist nur in zwei gegenüberliegenden Richtungen entsprechend dem Bezugszeichen 128 ausgestaltet und einer ovalen oder elliptische Aufnahme entspricht, so dass sich der Mutternkopf 117 bezüglich dem Klemmelement 180 nicht verdrehen kann. Somit kann bei einem Festlegen der Orientierung der Backen 11 und 12 für die Achsen zur Aufnahme der Stäbe sichergestellt werden, dass die Mutter 116 bei einem Anziehen der Schraube 103 nicht durchdreht. Andererseits erlaubt die Mutter 116 Verkippungen des Klemmelementes 180 gegenüber ihrer Lage, wie es auch die gegenüberliegende Schraube 103 dem zugehörigen Klemmelement gestattet.

Der Durchmesser der Verdrehsicherung 90 beträgt 30 Millimeter und die Auflagefläche (radiale Breite) für den äusseren Kranz 94 beträgt 3 Millimeter. Anstelle eines Bauelementes 90 können die Strukturen (Erhöhungen) auch im Material der Klemmbacke 13 eingebracht sein, beispielsweise radiale Rillen.

Die Rippen 21 der Klemmbacken 12 sind insbesondere dafür vorgesehen, um die halbe Kraft in vertikaler Richtung, das heisst in Längsrichtung der Schraube 103, als Druckkraft in das Federelement, das heisst das Scharniermittel 17 bzw. 33 einzuleiten.

Anstelle eines Halbzylinders 36 und komplementäre Rille 38 können beispielsweise auch ebene gegenüberliegende Flächen zum Einsatz kommen. Diese gegenüberliegende Flächen sollten nur in der Ruheposition des Klemmelementes weniger weit voneinander entfernt angeordnet sein als der Schlitzabstand 27. Komplementäre Verschwenkanschläge 36, 38 sind in diesem Sinne auch zwei erhabene Rippen auf den Klemmbacken 12 und 13, da diese ebenfalls einen Schwenkhebel bilden. Die Scharniermittel des dritten und vierten Ausführungsbeispiels sind damit in federnde Stege 33, 34 und 35 sowie Schwenkhebel bildende Anschlagmittel 36, 38 aufgeteilt. Solche Verschwenkanschläge können auch bei den Klemmelementen des ersten, zweiten und fünften Ausführungsbeispiels vorgesehen sein.

Statt einer Schraube 103 kann auch ein anderes Verriegelungselement vorgesehen sein, beispielsweise ein Spannhebel oder ein Bajonettverschluss.

Es wird hervorgehoben, dass die Bezeichnung Ausführungsbeispiel in der vorgenannten Beschreibung nicht bedeutet, dass nur die zu dem jeweiligen Klemmelement oder Gelenkelement beschriebenen Elemente Gegenstand der Erfindung sind. Es sind dies insbesondere auch Kombinationen aus Merkmalen, die in Gegenständen verschiedener Fig. beschrieben sind. So ist - als Beispiel - auch ein Klemmelement Gegenstand der Erfindung, dass die Bohrung und Mutter nach Fig. 38, die ausladenden Federbügel 33 nach Fig. 27 bis 31, eine Kontermutter 109 nach Fig. 15 und Rutschsicherungselemente 99 für die Stäbe nach Fig. 32 aufweist oder eine Teilmenge davon, insofern als solche Kombinationen sich den Ansprüchen unterordnen Lassen. Ein entsprechendes Gelenkelement kann aus zwei beliebigen vorstehenden Klemmelementen aufgebaut sein, sofern diese für die gewählte Verdrehsicherung einsetzbar sind.

### Bezugszeichen

- 10: Klemmelement (erstes Ausführungsbeispiel)
- 11: Hohlraum
- 12: Klemmbacke
- 13: Klemmbacke
- 14: quer verlaufende Nut
- 15: freies Ende
- 16: Aussenkante
- 17: Scharniermittel
- 18: Ausnehmung
- 19: Materialquerschnitt
- 20: Klemmelement(zweites Ausführungsbeispiel)
- 21: Querrippen
- 22: Ringflansch
- 23: Schraubenaufnahme, konische Schulter
- 24: ringförmigen Absatz
- 25: Ausnehmung
- 26: Bohrung
- 27: Schlitz
- 28: abgerundeter Bereich
- 29: Kotflügel
- 30: Klemmelement (drittes Ausführungsbeispiel)
- 31: Bohrung
- 32: Führungsrippe
- 33: Verbindungssteg
- 34: kurzer Übergangssteg
- 35: langer Steg
- 36: Halbzylinder
- 37: Schlitz
- 38: komplementäre Rille
- 39: Schlitz
- 40: Klemmelement (viertes Ausführungsbeispiel)
- 41: Wandung
- 42: Übergangsstufe
- 50: Klemmelement (fünftes Ausführungsbeispiel)
- 60: Klemmelement (sechstes Ausführungsbeispiel)
- 70: Klemmelement (siebtes Ausführungsbeispiel)
- 80: Klemmelement (achtes Ausführungsbeispiel)
- 90: Verdrehsicherung
- 91: Bohrung
- 92: Nabe
- 93: Speiche
- 94: äusserer Kranz
- 95: Erhöhung
- 96: Vertiefung
- 97: Scharnierelement
- 98: kleinerer Abstand
- 99: Rutschsicherungselement
- 100: Gelenkelement (erstes Ausführungsbeispiel)
- 101: dünnes stabförmige Element
- 102: dickes stabförmige Element
- 103: Schraube
- 104: konischer Flansch, Schulter
- 105: Vierkant, Sechskant oder Schlitz
- 106: Mutter
- 107: konische Hülse
- 108: konischer Flansch
- 109: Sicherungsschraube
- 110: Gelenkelement (zweites Ausführungsbeispiel)
- 111: schmalerer Abschnitt
- 112: breiter zweiter Abschnitt
- 113: Schulter
- 114: Innengewinde
- 116: Mutter
- 117: Mutternkopf
- 118: grösserer Abstand
- 119: Spiralfeder
- 120: Gelenkelement (drittes Ausführungsbeispiel)
- 121: Federaufnahme
- 122: Mutternaufnahme
- 123: Mutternkopfrückseite
- 124: Hülse
- 125: kleinerer Durchmesser
- 126: Bohrung
- 128: elliptische Aufnahme
- 130: Gelenkelement (viertes Ausführungsbeispiel)
- 131: Bohrung
- 140: Gelenkelement (fünftes Ausführungsbeispiel)
- 150: Gelenkelement (sechstes Ausführungsbeispiel)
- 160: Gelenkelement (siebtes Ausführungsbeispiel)
- 180: Klemmelement (neuntes Ausführungsbeispiel)
- 190: Verdrehsicherung
- 191: radiales Prisma
- 195: Mantelfläche
- 196: konische Abschrägungen
- 197: Boden- und Deckelfläche
- 198: zentrale Bohrung
- 199: Kunststoffschaumelement

## Patentansprüche

1. Einstückiges Klemmelement (10, 20, 30, 40, 50, 60, 70, 80, 180), das über zwei gegenüberliegende und einen seitlich offenen Hohlraum (11) zur Aufnahme eines stabförmigen Elementes (101, 102) bildende Klemmbacken (12, 13) und ein Scharniermittel (33, 34, 35, 36, 38; 97) verfügt, welches gegenüber dem Hohlraum (11) angeordnet ist, die Klemmbacken (12, 13) miteinander verbindet und **dadurch** diese aufeinander zu bewegbar sind, wobei jede Klemmbacke (12, 13) jeweils eine Bohrung (26, 31) aufweist, die miteinander fluchtend ausgerichtet sind, wobei das Scharniermittel (33, 34, 35, 36, 38; 97) federnd ist und die Bohrungen (26, 31) zwischen Hohlraum (11) und Scharniermittel (33, 34, 35, 36, 38; 97) angeordnet sind, **dadurch gekennzeichnet, dass** die Scharniermittel (33, 34, 35, 36, 38; 97) über komplementäre Verschwenkanschläge (36 bzw. 38) verfügen, die ab einer vorbestimmten Verformung von einem dem Hohlraumgegenüberliegenden, oder zwei seitlichen, federnden Verbindungsstegen (33, 34, 35, 97) in einen Eingriff kommen, und die ersten und zweiten Klemmbacken (13, 12) um eine durch sie vorgegebene Achse verschwenken.

2. Klemmelement nach Anspruch 1, **dadurch gekennzeichnet, dass** die erste Klemmbacke (13) an ihrer Aussenseite über eine Verdrehsicherung (190) oder über eine Aufnahme (24) für eine Verdrehsicherung (90, 199) verfügt.

3. Klemmelement nach Anspruch 1 oder Anspruch 2, **dadurch gekennzeichnet, dass** die zwei federnde seitliche Stege (33, 34, 35) jeweils in die ersten und zweiten Klemmbacken (13, 12) übergehen.

4. Klemmelement nach Anspruch 3, **dadurch gekennzeichnet, dass** die seitlichen Stege (33, 34, 35) ein halbkreisförmiges Mittelstück umfassen, an das sich beim Übergang in die erste Klemmbacke (13) ein Übergangssteg (34) anschliesst und an das sich beim Übergang in die zweite Klemmbacke (12) ein längerer Übergangssteg (35) anschliesst.

5. Klemmelement nach Anspruch 4, **dadurch gekennzeichnet, dass** der längere Steg (35) im Bereich der Wand (41) benachbart zum Hohlraum (11) als Rippe mit der zweiten Klemmbacke (12) verbunden ist.

6. Klemmelement nach einem der Ansprüche 3 bis 5, **dadurch gekennzeichnet, dass** die Stege (33, 34, 35) durch einen Schlitz (39) seitlich von den Klemmbacken (12, 13) getrennt sind.

7. Klemmelement nach Anspruch 1 oder Anspruch 2, **dadurch gekennzeichnet, dass** der federnde dem Hohlraum (11) gegenüberliegende Steg im wesentlichen C-förmigen Steg (97) ist und jeweils in die ersten und zweiten Klemmbacken (13, 12) übergeht.

8. Klemmelement nach einem der vorstehenden Ansprüche 1 bis 7, **dadurch gekennzeichnet, dass** die Bohrung (26) in der ersten Klemmbacke (13) zylindrisch ist und drei oder mehr longitudinale Ausrichtungsrippen (32) aufweist.

9. Klemmelement nach einem der vorstehenden Ansprüche 1 bis 8, **dadurch gekennzeichnet, dass** die Bohrung (31) in der zweiten Klemmbacke (12) in Richtung der ersten Klemmbacke (13) konisch verjüngend zuläuft und einen im Durchmesser grösseren Stufenabsatz (23) zur Aufnahme eines Kopfes einer Schraube (103) oder einer Mutter (106, 116) aufweist.

10. Klemmelement nach Anspruch 9, **dadurch gekennzeichnet, dass** die Bohrung (31) eine seitliche Ausnehmung (131) zur Aufnahme einer Verdrehsicherung (117) der Mutter (116) aufweist.

11. Gelenkelement (100, 110, 120, 130, 140, 150, 160)
- mit zwei Klemmelementen (10, 20, 30, 40, 50, 60, 70, 80, 180) nach einem der Ansprüche 1 bis 10, die mit ihren ersten Klemmbacken (13) aufeinander ausgerichtet übereinander angeordnet sind,
- mit einem mindestens zweiteiligen Verriegelungselement (103, 106, 109), wobei ein erstes Teil (103) des Verriegelungselementes durch die Bohrung (31) der zweiten Klemmbacke (12) des einen Klemmelementes einführbar ist, wobei ein zweites Teil (106) des Verriegelungselementes durch die Bohrung (31) der ersten Klemmbacke (12) des anderen Klemmelementes einführbar ist, wobei das eine oder das andere oder beide Teile (103, 106) durch die Bohrungen (26) in den ersten Klemmbacken (13) miteinander in Kontakt bringbar sind und wobei mit dem Verriegelungselement (103, 106) die Klemmbacken (12, 13) der Klemmelemente blockierbar sind.

12. Gelenkelement nach Anspruch 11, **dadurch gekennzeichnet, dass** zwischen den aufeinander ausgerichtet übereinander angeordneten ersten Klemmbacken (13) eine Verdrehsicherung (90, 199, 119) angeordnet ist, die eine zentrale Bohrung (91, 198) aufweist.

13. Gelenkelement nach Anspruch 12, **dadurch gekennzeichnet, dass** die Verdrehsicherung (90) ein Plättchen ist, dessen Material vorteilhafterweise härter als das Material der Klemmelemente ist, und/oder das bevorzugterweise auf beiden Seiten über Erhebungen (95) verfügt, insbesondere über gestanzte Erhebungen (95).

14. Gelenkelement nach Anspruch 12, **dadurch gekennzeichnet, dass** die Verdrehsicherung (199) ein Zylinder ist, dessen Material im Boden- und Deckelbereich vorteilhafterweise härter als das Material der Klemmelemente ist, und/oder das bevorzugterweise im Vollmaterialteil aus einem flexiblen, zusammendrückbaren Material, insbesondere einem Kunststoffschaum besteht.

15. Gelenkelement nach einem der Ansprüche 11 bis 14, **dadurch gekennzeichnet, dass** das Verriegelungselement (103, 106) eine zylindrische Schraube (103) und eine konische Mutter (106) umfasst, und dass vorteilhafterweise die konische Mutter (106) über eine Anschlagschulter (113) für eine Sicherungsschraube (109) verfügt, die in ein in der zylindrischen Schraube (103) vorgesehenem Innengewinde (114) einsetzbar ist.

16. Gelenkelement nach einem der Ansprüche 11 bis 15, **dadurch gekennzeichnet, dass** als Verdrehsicherung (119) oder als zusätzliche Verdrehsicherung (119+90; 119+199) eine hohle, das Verriegelungselement (103, 106) umfassende Feder vorgesehen ist, die die beiden Klemmelemente voneinander weg drückt.

## Claims

1. Clamping element (10, 20, 30, 40, 50, 60, 70, 80, 180) made in one piece, which clamping element comprises two clamping jaws (12, 13), which are arranged on opposite sides and which form a laterally open hollow space (11) to receive a rod shaped element (101, 102) and a hinge means (33, 34, 35, 36, 38; 97), which is arranged on the opposite side of the hollow space (11), which connects the clamping jaws (12, 13) with each other, whereby these are movable towards each other, wherein each clamping jaw (12, 13) has a borehole (26, 31), which are aligned with each other, wherein the hinge means (33, 34, 35, 36, 38; 97) is resilient and the boreholes (26, 31) are arranged between the hollow space (11) and the hinge means (33, 34, 35, 36, 38; 97), **characterized in that** the hinge means (33, 34, 35, 36, 38; 97) comprise complementary pivoting blocks (36 or 38), which engage if a predetermined deformation of one connection bridge (33, 34, 35, 97) arranged on the opposite side of the hollow space (11) or of two lateral, resilient connection bridges (33, 34, 35, 97) is exceeded, and pivot the first and the second clamping jaws (12, 13) about an axis given by them.

2. Clamping element according to claim 1, **characterized in that** the first clamping jaw (13) comprises on its exterior a twist protection (190) or a receiver (24) for a twist protection (90, 199).

3. Clamping element according to claim 1 or claim 2, **characterized in that** the two resilient lateral bridges (33, 34, 35) merge into the first and the second clamping jaws (13, 12), respectively.

4. Clamping element according to claim 3 **characterized in that** the lateral bridges (33, 34, 35) comprise a semi circle shaped centrepiece, on which a transition bridge (34) is attached at the transition into the first clamping jaw (13) and on which a longer transition bridge (35) is attached at the transition into the second clamping jaw (12).

5. Clamping element according to claim 4, **characterized in that** the longer bridge (35) is connected to the second clamping jaw (12) as a rib in the area of the wall (41) adjacent to the hollow space (11).

6. Clamping element according to one of claims 3 to 5, **characterized in that** the bridges (33, 34, 35) are separated laterally from the clamping jaws (12, 13) by a slit (39).

7. Clamping element according to claim 1 or claim 2, **characterized in that** the resilient bridge arranged on the opposite side of the hollow space (11) is substantially a C-shaped bridge (97), and merges into the first and the second clamping jaws (13, 12), respectively.

8. Clamping element according to one of the preceding claims 1 to 7, **characterized in that** the borehole (26) is cylindrical in the first clamping jaw (13) and has three or more longitudinal alignment ribs (32).

9. Clamping element according to one of the preceding claims 1 to 8, **characterized in that** the borehole (31) in the second clamping jaw (12) is conically tapered towards the first clamping jaw (13) and has a level section (23), which is bigger in diameter, to receive the head of a screw (103) or a nut (106, 116).

10. Clamping element according to claim 9, **characterized in that** the borehole (31) has a lateral recess (131) to receive a twist protection (117) of the nut (116).

11. Joint element (100, 110, 120, 130, 140, 150, 160)
- with two clamping elements (10, 20, 30, 40, 50, 60, 70, 80, 180) according to one of claims 1 to 10, which are aligned with each other with their first clamping jaws (13) and arranged on top of each other,
- with a locking element (103, 106, 109), which is made of at least two pieces, wherein a first part (103) of the locking element is insertable through the borehole (31) of the second clamping jaw (12) of one of the clamping elements, wherein a second part (106) of the locking element is insertable through the borehole (31) of the first clamping jaw (12) of the other clamping element, wherein the one or the other or both parts (103, 106) are engageable with each other through the boreholes (26) in the first clamping jaw (13) and wherein the clamping jaws (12, 13) of the clamping elements are blockable with the locking element (103, 106).

12. Joint element according to claim 11, **characterized in that** between the first clamping jaws (13), which are aligned with each other and arranged on top of each other, a twist protection (90, 199, 119) is arranged, which comprises a central borehole (91, 198).

13. Joint element according to claim 12, **characterized in that** the twist protection (90) is a small plate, the material of which is favourably harder than the material of the clamping elements, and/or which preferably has elevations (95) on both sides, particularly has punched elevations (95).

14. Joint element according to claim 12, **characterized in that** the twist protection (199) is a cylinder, the material of which is favourably harder in the area of the bottom and the lid than the material of the clamping elements, and/or which preferably consists of a flexible, compressible material, particularly a plastic foam.

15. Joint element according to one of claims 11 to 14, **characterized in that** the joint element (103, 106) comprises a cylindrical screw (103) and a conical nut (106), and that the conical nut (106) preferably has a blocking shoulder (113) for a security screw (109), which is insertable in an internal thread (114) provided in the cylindrical screw (103).

16. Joint element according to one of claims 11 to 15, **characterized in that** a hollow spring, which encompasses the locking element (103, 106) and which pushes the two clamping elements away from each other, is provided as twist protection (119) or as additional twist protection (119+90; 119+199).

## Revendications

1. Elément de serrage (10, 20, 30, 40, 50, 60, 70, 80, 180) d'une seule pièce, lequel comprend deux mâchoires (12, 13) de serrage qui sont arrangées sur des faces opposées et qui forment une cavité latéralement ouverte (11) pour recevoir un élément (101, 102) en forme de barre et un moyen de charnière (33, 34, 35, 36, 38; 97) qui est arrangé sur la face opposée de la cavité (11), qui connecte les mâchoires (12, 13) de serrage l'une avec l'autre, où elles peuvent être déplacées l'une vers l'autre, où chaque mâchoire (12, 13) de serrage comprend un alésage (26, 31), qui sont alignés l'un avec l'autre, où le moyen de charnière (33, 34, 35, 36, 38; 97) est élastique et où les alésages (26, 31) sont arrangés entre la cavité (11) et le moyen de charnière (33, 34, 35, 36, 38; 97), **caractérisé en ce que** les moyens de charnière (33, 34, 35, 36, 38; 97) possèdent deux butoirs complémentaires (36 ou 38), qui interviennent si une déformation prédéterminée d'un pont de connexion (33, 34, 35, 97) arrangé sur la face opposée de la cavité (11) ou de deux ponts de connexion élastiques latéraux (33, 34, 35, 97) est dépassée et pivotent les premières et deuxièmes mâchoires (12, 13) de serrage autour d'un axe donné par ceux-ci.

2. Elément de serrage selon la revendication 1, **caractérisé en ce que** la première mâchoire de serrage (13) comprend, à sa face extérieure, une protection anti-rotation (190) ou une réception (24) pour une protection anti-rotation (90, 199).

3. Elément de serrage selon la revendication 1 ou la revendication 2, **caractérisé en ce que** les deux ponts élastiques latéraux (33, 34, 35) s'intègrent dans les premières et les deuxièmes mâchoires (13, 12) de serrage.

4. Elément de serrage selon la revendication 3, **caractérisé en ce que** les ponts latéraux (33, 34, 35) comprennent une pièce centrale en forme d'un demi-cercle, à la quelle un pont de transition (34) est attaché à la transition dans la première mâchoire (13) de serrage et à la quelle un pont de transition (35) plus long est attaché à la transition dans la deuxième mâchoire (12) de serrage.

5. Elément de serrage selon la revendication 4, **caractérisé en ce que** le pont (35) plus long est connecté avec la seconde mâchoire (12) de serrage en tant que côte dans la zone de la paroi (41) adjacente à la cavité (11).

6. Elément de serrage selon l'une des revendication 3 à 5, **caractérisé en ce que** les ponts (33, 34, 35) sont séparés par une fente (39) des mâchoires (12, 13) de serrage.

7. Elément de serrage selon la revendication 1 ou 2, **caractérisé en ce que** le pont élastique arrangé à l'opposé de la cavité (11) est essentiellement un pont en forme de C (97) et **en ce qu'**il s'intègre dans les premières et deuxièmes mâchoires (13, 12) de serrage.

8. Elément de serrage selon l'une des revendications 1 à 7, **caractérisé en ce que** l'alésage (26) dans la première mâchoire (30) de serrage est cylindrique et comprend trois ou plus côtes d'alignement (32) longitudinales.

9. Elément de serrage selon l'une des revendications 1 à 4, **caractérisé en ce que** l'alésage (31) dans la deuxième mâchoire (12) de serrage s'amincit d'une manière conique dans la direction de la première mâchoire (13) de serrage et comprend une section de pas (23) avec un diamètre plus grand pour recevoir la tête d'une vis (103) ou d'un écrou (106, 116).

10. Elément de serrage selon la revendication 9, **caractérisé en ce que** l'alésage (31) possède un évidement (131) latéral pour recevoir une protection anti-rotation (117) de l'écrou (116) .

11. Elément de joint (100, 110, 120, 130, 140, 150, 160)
- avec deux éléments de serrage (10, 20, 30, 40, 50, 60, 70, 80, 180) selon l'une des revendications 1 à 10, qui sont alignés avec leur premières mâchoires (13) de serrage et qui sont arrangées l'une sur l'autre,
- avec un élément de verrouillage (103, 106, 109) qui est fait en au moins deux pièces, où une première pièce (103) de l'élément de verrouillage peut être introduite à travers l'alésage (31) de la deuxième mâchoire (12) de serrage de l'un des éléments de serrage, où une deuxième pièce (106) de l'élément de verrouillage peut être introduite à travers l'alésage (31) de la première mâchoire (12) de serrage de l'autre élément de serrage, où l'une ou l'autre ou les deux pièces (103, 106) peuvent s'engager mutuellement à travers les alésages (26) dans les premières mâchoires (13) de serrage et où les mâchoires (12, 13) de serrage des éléments de serrage peuvent être bloquées avec l'élément de verrouillage (103, 106).

12. Elément de joint selon la revendication 11, **caractérisé en ce qu'**entre les deux premières mâchoires (13) de serrage, qui sont alignées l'une avec et sur l'autre, une protection anti-rotation (90, 199, 119) est arrangée, qui comprend un alésage (91, 198) central.

13. Elément de joint selon la revendication 12, **caractérisé en ce que** la protection anti-rotation (90) est une petite plaque, dont le matériel est préférablement plus dur que le matériel des éléments de serrage, et/ou possède préférablement des élévations (95) sur les deux côtés, en particulier des élévations (95) estampées.

14. Elément de joint selon la revendication 12, **caractérisé en ce que** la protection anti-rotation (199) et un cylindre, dont le matériel est préférablement plus dur dans sa zone inférieure et supérieure que le matériel des éléments de serrage et/ou il consiste préférablement d'un matériel compressible flexible, en particulier une mousse plastique.

15. Elément de joint selon l'une des revendications 11 à 14, **caractérisé en ce que** l'élément de joint (103, 106) comprend une vis (103) cylindrique et un écrou (106) conique et **en ce que** l'écrou (106) conique possède préférablement une épaule (113) de butoir pour une vis (109) de sécurité, qui peut être introduit dans un filetage (114) intérieur prévu dans la vis (103) cylindrique.

16. Elément de joint selon l'une des revendications 11 à 15, **caractérisé en ce que** un ressort creux, qui est arrangé autour de l'élément de verrouillage (103, 106) et qui repousse les deux éléments de serrage l'un de l'autre, est prévu en tant que protection anti-rotation (119) ou en tant que protection anti-rotation supplémentaire (119+90 ; 119+199).
